(19) Europäisches Patentamt
European Patent Office
Office européen des brevets

(11) EP 1 714 638 A1

(12) DEMANDE DE BREVET EUROPEEN

(43) Date de publication:
**25.10.2006 Bulletin 2006/43**

(21) Numéro de dépôt: **06110830.4**

(22) Date de dépôt: **08.03.2006**

(51) Int Cl.:
***A61K 8/368*** *(2006.01)* ***A61Q 15/00*** *(2006.01)*
***A61K 8/26*** *(2006.01)* ***A61K 8/27*** *(2006.01)*

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **19.04.2005 FR 0550991**

(71) Demandeur: **L'ORÉAL**
**75008 Paris (FR)**

(72) Inventeurs:
- **Lemoine, Cyril**
**95380 Puiseux-en-France (FR)**
- **Masson-Deblaize, Anne**
**94100 Saint Maur (FR)**

(74) Mandataire: **Miszputen, Laurent**
**L'OREAL - D.I.P.I.**
**25-29 Quai Aulagnier**
**92600 Asnières (FR)**

# (54) Composition cosmetique deodorante comprenant l'association du salicylate de zinc ou l'un de ses derives et d'un sel d'aluminium anti-transpirant

(57) L'invention a pour objet une composition cosmétique déodorante comprenant au moins :
a) le salicylate de zinc ou l'un de ses dérivés, sous forme libre ou hydratée et
b) au moins un sel d'aluminium anti-transpirant ; le rapport en poids salicylate de zinc /sel d'aluminium allant de 1/500 à 10/1 et de préférence de 1/200 à 1/1.

L'invention concerne également un procédé de traitement cosmétique de la transpiration humaine et des odeurs axillaires humaines à l'aide de cette composition.

EP 1 714 638 A1

## Description

L'invention a pour objet une composition cosmétique déodorante comprenant au moins :

a) le salicylate de zinc ou l'un de ses dérivés, sous forme libre ou hydratée et
b) au moins un sel d'aluminium anti-transpirant ; le rapport en poids salicylate de zinc /sel d'aluminium allant de 1/500 à 10/1 et de préférence de 1/200 à 1/1.

L'invention concerne également un procédé de traitement cosmétique de la transpiration humaine et des odeurs axillaires humaines à l'aide de cette composition.
L'invention concerne l'utilisation de l'association du salicylate de zinc ou de l'un de ses dérivés et d'un sel d'aluminium anti-transpirant comme actif déodorant dans une composition cosmétique.
Dans le domaine cosmétique, il est bien connu d'utiliser en application topique, des produits déodorants contenant des substances actives de type anti-transpirant de type bactéricide ou du type absorbeur d'odeurs pour diminuer, voire supprimer, les odeurs axillaires généralement désagréables.
Les substances bactéricides inhibent le développement de la flore cutanée responsable des odeurs axillaires. Ils ont pour inconvénient de ne pas être actifs sur l'odeur de la sueur déjà développée. Parmi les produits bactéricides, le plus couramment employé est le Triclosan (2,4,4'-trichloro-2'-hydroxydiphényléther), qui présente l'inconvénient de modifier de façon importante l'écologie de la flore cutanée, d'être inhibé par certains composés, comme par exemple les tensio-actifs non-ioniques, couramment utilisés dans la formulation de compositions cosmétiques. Par ailleurs, le caractère insoluble du Triclosan dans l'eau ne permet pas son incorporation dans des formules essentiellement aqueuses.
Les substances anti-transpirantes ont pour effet de limiter le flux sudoral. Elles sont généralement constituées de sels d'aluminium. Leur efficacité déodorante est limitée lorsqu'ils sont utilisés seuls. De plus, dans des concentrations élevées, ces substances présentent un potentiel irritant pour la peau.
Parmi les actifs déodorants, certains sels hydrosolubles de zinc ont déjà été proposés dans des produits déodorants comme par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc. De telles formulations déodorantes ont été notamment décrites dans les documents WO93/01793, EP468564, EP024176 et EP768080.
Dans les années 1930 comme le décrit en particulier l'ouvrage Drugs & Cosmetic Industry-Modern Cosmetics ; Chapître Déodorants pages 168-177 (1934), on savait que le salicylate de zinc pouvait être formulé dans des formulations déodorantes du type pâte ou sous forme de stick en présence nécessaire d'autres actifs pour une action déodorante suffisante. Il a été proposé plus récemment comme actif déodorant possible dans différents supports : selon le brevet EP318206 dans des gels sticks à base de benzylidene sorbitol, d'un émollient du type di-isopropyladipate, d'un ou plusieurs solvants polaires et d'un agent de couplage ; selon les brevets US 5,254,332 et US 5,302,381 dans des sticks contenant un huile siliconée volatile, une cire de bas point de fusion, un émollient insoluble dans l'eau liquide et en présence d'un actif déodorant du type bicarbonate de sodium ; selon la demande WO02/13776 dans des gels aqueux à base de savons d'acides gras.
Cependant l'efficacité de ses sels de zinc est limitée lorsqu'ils sont utilisés seuls.
Pour permettre d'augmenter l'efficacité déodorante, différentes associations d'actifs ont été proposées.
Dans la demande de brevet WO97/24131, on a proposé d'associer le salicylate de zinc avec un acylglycine en $C_6$-$C_{10}$.
On connaît également dans le brevet US 6,426,061 des compositions pour combattre le développement des odeurs de la transpiration de la peau humaine comprenant l'association des actifs suivants : (1) un inhibiteur de récepteur androgène (resvératrol, epigallocatéchine-3-gallate ou acide flufénamique) ; (2) un actif anti-DHT (sels de zinc en particulier le sulfate de zinc) (3) un inhibiteur de protéines porteuses d'odeurs, (4) un sel d'aluminium anti-transpirant et (5) un agent antimicrobien du type chlorhexidine digluconate ou chlorhexidine diacétate. Ces compositions présentent l'inconvénient d'utiliser des agents antimicrobiens particulièrement actifs sur la flore cutanée.
On connaît dans la demande de brevet WO01/52804 des compositions déodorantes à base de sels antitranspirants auxquels on propose de rajouter des chélatants de métaux de transition (DPTA). Ces formulations sont potentiellement écotoxiques.
Dans la demande de brevet EP1486199, on a décrit des compositions aérosols alcooliques à base de salicylate de zinc ou de l'un de ses dérivés. Bien que soit décrite de manière » génarale, dans cette demande, la possibilité d'associer des sels dantitranspirants, la demanderesse a constaté que dans des quantités relatives particulières l'efficacité déodorante obtenue était insuffisante.
Le but de la présente invention est donc de disposer de nouvelles compositions cosmétiques comprenant un système déodorant ayant une efficacité supérieure à celle des sels anti-transpirants et à celle des sels de salicylate de

zinc utilisés seuls et qui ne présentent pas les inconvénients des produits déodorants de l'art antérieur tel qu'évoqués précédemment.

La demanderesse a découvert qu'une telle composition est obtenue en utilisant l'association du salicylate de zinc ou l'un de ses dérivés et d'un sel d'aluminium anti-transpirant dans un rapport en poids salicylate de zinc /sel d'aluminium allant de 1/500 à 10/1 et de préférence de 1/200 à 1/1. La demanderesse a découvert de manière inattendue que cette association particulière présentait un effet de synergie au niveau de la diminution de l'intensité de l'odeur par rapport aux actifs utilisés individuellement.

De façon plus précise, l'invention a pour objet une composition cosmétique déodorante comprenant au moins :

a) le salicylate de zinc ou l'un de ses dérivés, sous forme libre ou hydratée et

b) au moins un sel d'aluminium anti-transpirant ; le rapport en poids salicylate de zinc /sel d'aluminium allant de 1/500 à 10/1 et de préférence de 1/200 à 1/1.

L'invention concerne également un procédé de traitement cosmétique de la transpiration humaine et des odeurs axillaires humaines à l'aide de cette composition.

L'invention concerne l'utilisation de l'association du salicylate de zinc ou de l'un de ses dérivés et d'un sel d'aluminium anti-transpirant comme actif déodorant dans une composition cosmétique ; dans un rapport en poids salicylate de zinc /sel d'aluminium allant de 1/500 à 10/1 et de préférence de 1/200 à 1/1.

Au sens de la présente invention, on entend par "composition déodorante" toute composition capable de réduire le flux sudoral, masquer, absorber, améliorer et/ou réduire l'odeur désagréable résultant de la décomposition de la sueur humaine par des bactéries.

Par " sel d'aluminium anti-transpirant", on entend tout sel ou tout complexe d'aluminium ayant pour effet de diminuer ou limiter le flux sudoral.

Les sels d'aluminium conformes à l'invention sont choisis de préférence parmi les halohydrates de d'aluminium ; les halohydrates d'aluminium et de zirconium, les complexes d'hydroxychlorure de zirconium et d'hydroxychlorure d'aluminium avec ou sans un acide aminé tels que ceux décrits dans le brevet US-3792068.

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Les sels d'aluminium anti-transpirants peuvent être présents dans la composition selon l'invention à raison d'environ 0,5 à 25% en poids par rapport au poids total de la composition.

Le salicylate de zinc et ses dérivés selon l'invention répondent en général à la structure suivante :

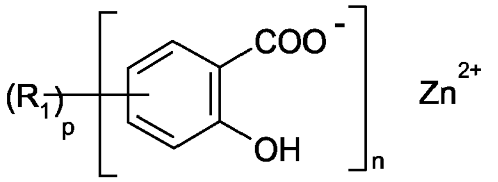

(I)

dans laquelle n = 2, p vaut 0, 1, 2 ou 3 ; $R_1$ désigne un alkyle en $C_1$-$C_{18}$ linéaire ou ramifié (par exemple méthyle, éthyle, n-propyle, isopropyle, n-butyle); un radical hydroxyalkyle en $C_1$-$C_{18}$, linéaire ou ramifié ; un atome d'halogène (par exemple Iode, Brome, Chlore) ; un radical acyle en $C_2$-$C_{18}$ (par exemple acétyle) ; un radical $COR_2$, $OCOR_2$ ou $CONHR_2$ où $R_2$ désigne un atome d'hydrogène ou un radical alkyle en $C_1$-$C_{18}$ linéaire ou ramifié.

On choisira plus préférentiellement le salicylate de zinc (p = 0) et plus particulièrement sous forme hydratée comme le salicylate de zinc trihydraté tel que le produit commercial vendu sous le "Zinc Salicylate Trihydrate" par la

société Bernardy Chimie .

Dans les compositions de l'invention, la concentration en salicylate de zinc varie de préférence de 0,001 à 20%, plus préférentiellement de 0,01 à 15% et encore plus préférentiellement de 0,05 à 5% en poids par rapport au poids total de la composition.

Les compositions déodorantes selon l'invention destinées à l'usage cosmétique peuvent se présenter sous forme de lotions, de crèmes ou de gels fluides distribués distribués en spray aérosol, en flacon pompe ou en roll-on, sous forme de crèmes épaisses distribuées en tubes ou en grille ; sous forme de bâtonnets (sticks), et contenir à cet égard les ingrédients généralement utilisés dans ce type de produits et bien connus de l'homme de l'art, sous réserve qu'ils n'interfèrent pas avec le sel d'aluminium et le salicylate de zinc décrit dans la présente invention.

De préférence, on évitera de conditionner les compositions selon l'invention dans un dispositif aérosol alcoolique dans la mesure où le mélange sel d'aluminium/alcool en $C_1$-$C_4$/conditionnement aérosol a tendance à provoquer soit une dégradation de la composition soit une corrosion des bidons aérosols, soit une perte d'efficacité de l'actif antitranspirant. Selon une forme préférentielle, la composition est différente d'une composition aérosol alcoolique.

Par « composition aérosol alcoolique », on entend toute formulation comprenant au moins un agent propulseur et au moins un mono-alcool en $C_1$-$C_4$ comme le propanol, l'éthanol ou l'isopropanol.

Les compositions déodorantes selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse desdites compositions contient de l'eau et en général d'autres solvants solubles ou miscibles dans l'eau. Les solvants solubles ou miscibles dans l'eau comprennent les mono alcools à chaîne courte par exemple en $C_1$-$C_4$ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Selon une forme particulière de l'invention, les compositions anti-transpirantes peuvent être anhydres.

Par "anhydre", on entend au sens de l'invention, une composition dont la teneur en eau libre ou ajoutée est inférieure à 3% et de préférence dont la teneur en eau ajoutée est inférieure à 1% en poids par rapport au poids total de la composition.

Les compositions selon l'invention comprennent de préférence au moins une phase liquide organique non-miscible dans l'eau Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones $D_4$, $D_5$ ou $D_6$.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les huiles émollientes non-volatiles utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{18}$ et leurs mélanges, des polyols en $C_2$-$C_6$ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

La composition cosmétique déodorante selon l'invention peut contenir un ou plusieurs actifs déodorants additionnels comme par exemple des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels

d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 $\mu$m et masse volumique 40 kg/m3), 551 DE 20 (granulométrie d'environ 30 $\mu$m et masse volumique 65 kg/m3), 551 DE 50 (granulométrie d'environ 40 $\mu$m), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 $\mu$m et notamment allant de 0,02 $\mu$m à 1 $\mu$m, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans compositions déodorantes.

Les compositions selon l'invention peuvent également contenir également un ou plusieurs autres agents structurants ou gélifiants de la phase liquide organique non-miscible dans l'eau de la composition tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'—dibutylsuccinamide) ;les organopolysiloxane élastomères tels que ceux décrits dans la demande WO97/44010

La composition selon l'invention peut encore être pressurisée et être conditionnée dans un dispositif aérosol.

La présente invention a pour objet un dispositif aérosol constitué par :

(A) un récipient comprenant une composition déodorante telle que définie précédemment,

(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

La présente invention a également pour objet un procédé cosmétique pour traiter les odeurs axillaires humaines, consistant à appliquer sur la surface axillaire une quantité efficace d'une composition telle que définie ci-dessus.

Les exemples qui suivent servent à illustrer la présente invention.

**I/ Comparaison de l'activité déodorante de l'association salicylate de zinc/sel d'aluminium par rapport aux association triclosan/sel d'aluminium et ricinoleate de zinc /sel d'aluminium.**

Protocole du test d'efficacité déodorante

Des recueils de sueur axillaire sont effectués en sauna sur 6 volontaires, les échantillons de sueur individuelle, conservés dans la glace pendant quelques heures, sont alors pratiquement inodores. Ils sont ensuite mélangés et répartis en aliquots de 1 ml. Les actifs sont rajoutés à ces aliquots, puis mis à incuber à l'étuve 37°C. Après 24 heures d'incubation, un jury d'experts évalue l'intensité de l'odeur comparativement à un échantillon témoin : 1 ml de sueur incubée sans ajout d'actif. L'intensité est évaluée sur une échelle de 0 (pas d'odeur) à 4 (très forte odeur).

Les résultats sont exprimés en % de variation de l'intensité de l'odeur comparativement à cet échantillon de sueur témoin (moyenne des % de variation à T24h).

$$\Delta = \frac{\text{intensité odeur de l'échantillon témoin} - \text{intensité odeur de l'échantillon avec actif}}{\text{intensité odeur de l'échantillon témoin}} \times 100$$

| Actifs testés | Quantité testée (mg MA/ml de sueur | % de diminution de l'intensité de l'odeur |
|---|---|---|
| ACH | 0,1 mg MA | -35% |
| (A) | 0,1 mg MA | -21% |
| (B) | 0,4 mg MA | -49% |
| (C) | 0,3 mg MA | -4% |
| ACH + (A) | 0,2 mg MA | - 64% |
| ACH + (B) | 0,5 mg MA | -46% |

(suite)

| Actifs testés | Quantité testée (mg MA/ml de sueur | % de diminution de l'intensité de l'odeur |
|---|---|---|
| ACH + (C) | 0,4 mg MA | -38% |
| ACH : Aluminium chlorohydrate (Micro Dry - Reheis)<br>(A) : Salicylate de zinc (Bernardy Chimie)<br>(B) : Triclosan (CIBA)<br>(C) : Ricinoleate de zinc (Grillo Werke) | | |

On constate que l'association salicylate de zinc/sel d'aluminium (rapport en poids : 1/1) conduit à un effet de synergie au niveau de la diminution de l'intensité de l'odeur contrairement aux associations Triclosan/sel d'aluminium et ricinoléate de zinc/sel d'aluminium.

**Exemple 1: Stick déodorant**

| Ingrédients | Quantités |
|---|---|
| Cyclopentasiloxane (DC245 -DOW CORNING) | 32 g |
| PPG-14 Butyl ether (Ucon Fluid AP- Amerchol) | 10 g |
| Huile de ricin hydrogénée (Cutina HR- Cognis) | 4 g |
| Talc | 2 g |
| Aluminium Chlorhydrate (Micro Dry - Reheis) | 20 g |
| Salicylate de zinc (Bernardy Chimie) | 1 g |
| Alcool stéarylique | 14 g |
| PEG-8 distearate (Distéarate de PEG 400- Stéarines Dubois) | 2 g |
| $C_{12}$-$_{15}$ alkyl benzoate (Finsolv TN- Witco) | 15 g |
| | 100 |

On chauffe la cyclopentasiloxane à 65°C. On ajoute les autres ingrédients (1 par 1) en restant à 65-70°C. On homogénéise l'ensemble (solution transparente) pendant 15 minutes. On ajoute les 2 actifs déodorants et le talc. On refroidit à environ 55°C (quelques degrés Celcius au dessus de l'épaississement du mélange) et on coule dans les sticks. On met à 4°C pendant 30 minutes.

**Exemple 2 : Roll-on (émulsion)**

| Phase | Ingrédients | Quantités |
|---|---|---|
| A | Aluminium Chlorhydrate (50%solution) (Chlorhydrol 50% USP) | 40 g |
| | Salicylate de zinc (Bernardy Chimie) | 0,1 g |
| B | Steareth-21 (Brij 721-ICI) | 2g |
| | Steareth-2 (Brij 2 - ICI) | 2g |
| | Steareth-5 Stearate | 1g |
| | PPG-15 stearyl ether (Arlamol E - ICI) | 1,5g |
| | Cyclopentasiloxane (DC245 -DOW CORNING) | 3,5g |
| C | Eau | 49,9 g |

(suite)

| Phase | Ingrédients | Quantités |
|---|---|---|
| | | 100g |

On chauffe les phases (B) et (C) séparément à 70°C. On mélange (B) et (C) sous agitation Turax 5min puis refroidir à 55°C sous pâle. On ajoute A doucement en agitant. On homogénéise 1 à 3 minutes. On refroidit à 35°C sous agitation. La formule est stable 2 mois à 45°C.

**Exemple 3 : Spray non aérosol (Emulsion obtenue par inversion de phase)**

| Ingrédients | Quantités |
|---|---|
| Aluminium Chlorhydrate (50%solution) (Chlorhydrol 50% USP) | 20g |
| Salicylate de zinc (Bernardy Chimie) | 3g |
| Cetearyl isononanoate (and) Cetearyl alcohol (and) Ceteareth-20 (and) Glycerin (and) Glyceryl stearate (and) Ceteareth-12 (and) Cetyl palmitate (Emulgade CM- Cognis) | 15g |
| Eau | 62g |
| | 100g |

On solubilise le gluconate dans l'eau et ajouter à l'Emulgade CM sous agitation modérée. On ajoute la solution de sel d'aluminium sous agitation modérée. La formule est stable 2 mois à 45°C.

## Revendications

**1.** Composition cosmétique déodorante comprenant au moins :

a) le salicylate de zinc ou l'un de ses dérivés , sous forme libre ou hydratée ;
b) un sel d'aluminium anti-transpirant ;

le rapport en poids salicylate de zinc /sel d'aluminium allant de 1/500 à 10/1.

**2.** Composition selon la revendication 1, où le rapport en poids salicylate de zinc /sel d'aluminium varie de 1/200 à 1/1

**3.** Composition selon la revendication 1 ou 2, où le sel anti-transpirant est choisi parmi les halohydrates d'aluminium ; les halohydrates d'aluminium et de zirconium ; les complexes d'hydroxychlorure de zirconium et d'hydroxychlorure d'aluminium avec un acide aminé.

**4.** Composition selon la revendication 3, où le sel d'aluminium anti-transpirant est choisi parmi le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

**5.** Composition selon la revendication 3, où le sel d'aluminium anti-transpirant est choisi parmi l'aluminium zirconium octachlorohydrate, l'aluminium zirconium pentachlorohydrate, l'aluminium zirconium tetrachlorohydrate, l'aluminium zirconium trichlorohydrate .

**6.** Composition selon la revendication 3, où le sel d'aluminium anti-transpirant est choisi parmi les complexes d'hydroxychlorure de zirconium et d'hydroxychlorure d'aluminium avec la glycine.

**7.** Composition selon la revendication 6, où le sel d'aluminium anti-transpirant est choisi parmi les complexes aluminium zirconium octachlorohydrex glycine, aluminium zirconium pentachlorohydrex glycine, aluminium zirconium tetrachlorohydrex glycine et aluminium zirconium trichlorohydrex glycine.

**8.** Composition selon l'une quelconque des revendications précédentes, où le sel d'aluminium anti-transpirant est le chlorhydrate d'aluminium sous forme activée ou non activée.

**9.** Composition selon l'une quelconque des revendications 1 à 8, où le ou les sels d'aluminium anti-transpirants sont présents dans des quantités allant de 0,5 à 25% en poids par rapport au poids total de la composition.

**10.** Composition selon l'une quelconque des revendications 1 à 9, où le salicylate de zinc ou l'un de ses dérivés sont choisis parmi les composés de formule (I) suivante :

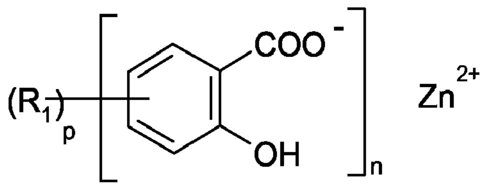

(I)

dans laquelle n = 2, p vaut 0, 1, 2 ou 3 ; R1 désigne un alkyle en C1-C18, linéaire ou ramifié ; un radical hydroxyalkyle en C1-C18, linéaire ou ramifié ; un atome d'halogène ; un radical acyle en $C_2$-$C_{18}$ ; un groupe $COR_2$, $OCOR_2$, $CONHR_2$ où $R_2$ désigne un atome d'hydrogène ou un radical alkyle en $C_1$-$C_{18}$, linéaire ou ramifié.

**11.** Composition selon la revendication 10, où le composé de formule (I) est le salicylate de zinc (p = 0).

**12.** Composition selon la revendication 11, où le composé de formule (I) est le salicylate de zinc trihydraté.

**13.** Composition selon l'une quelconque des revendications 1 à 12 , **caractérisée par le fait que** la concentration en salicylate de zinc varie de 0.001 à 20%, de préférence de 0,01 à 15% et encore préférentiellement de 0,05 à 5% en poids rapport au poids total de la composition.

**14.** Composition selon l'une quelconque des revendications 1 à 13, **caractérisée par le fait qu'**elle se présente sous forme de lotion, de crème ou de gel fluide distribué en spray aérosol, en flacon pompe ou en roll-on ; sous forme de crème ou gel distribué en tube ou en grille ; sous forme de bâtonnet (stick).

**15.** Composition selon l'une quelconque des revendications précédentes, **caractérisée par le fait qu'**elle est différente d'une composition aérosol alcoolique.

**16.** Composition selon l'une quelconque des revendications précédentes, **caractérisée par le fait qu'**elle comprend au moins une phase aqueuse.

**17.** Composition selon la revendication 16, **caractérisée par le fait qu'**elle se présente sous forme de lotion aqueuse, sous forme d'émulsion eau-dans-huile, huile-dans-eau ; sous forme d'émulsion multiple.

**18.** Composition selon la revendication 17, où la phase aqueuse contient de l'eau et un ou plusieurs solvants solubles ou miscibles dans l'eau.

**19.** Composition selon la revendication 18, où les solvants solubles ou miscibles dans l'eau sont choisis parmi les mono alcools en $C_1$-$C_4$ ; les diols ; les polyols.

**20.** Composition selon l'une quelconque des revendications 1 à 14, **caractérisée par le fait qu'**elle est anhydre.

**21.** Composition selon l'une quelconque des revendications 1 à 20, **caractérisée par le fait qu'**elle comprend au moins une phase liquide organique non-miscible dans l'eau.

**22.** Composition selon la revendication 21, dans laquelle la phase liquide organique comprend une ou plusieurs huiles

émollientes siliconée ou hydrocarbonée, volatiles ou non-volatiles.

**23.** Composition selon la revendication 22, dans laquelle les huiles émollientes sont présentes dans des quantités allant de 1 à 50% en poids et plus préférentiellement de 5 à 40% en poids par rapport au poids total de la composition.

**24.** Composition selon l'une quelconque des revendications 1 à 23, **caractérisée par le fait qu'**elle comprend en plus un ou plusieurs actifs déodorants additionnels.

**25.** Composition selon la revendication 24, **caractérisée par le fait qu'**elle comprend en plus un ou plusieurs agents bactériostatiques ou agents bactéricides.

**26.** Composition selon l'une quelconque des revendications 1 à 25, **caractérisée par le fait qu'**elle comprend en plus un ou plusieurs agents de suspension.

**27.** Composition selon l'une quelconque des revendications 1 à 26, **caractérisée par le fait qu'**elle comprend en plus au moins une poudre organique.

**28.** Composition selon l'une quelconque des revendications 1 à 27, **caractérisée par le fait qu'**elle comprend en plus au moins un additif cosmétique choisi parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs.

**29.** Composition selon l'une quelconque des revendications 21 à 28, **caractérisée par le fait qu'**elle comprend en plus un ou plusieurs agents structurants ou gélifiants de la phase liquide organique non-miscible dans l'eau.

**30.** Dispositif aérosol constitué par :

(A) un récipient comprenant une composition déodorante telle que définie selon l'une quelconque des revendications 1à 29 ;
(B) au moins un agent propulseur et un moyen de distribution de la dite composition aérosol.

**31.** Procédé cosmétique pour traiter les odeurs axillaires humaines, consistant à appliquer sur la surface axillaire une quantité efficace d'une composition telle que définie telle que définie selon l'une quelconque des revendications 1à 29.

**32.** Utilisation de l'association du salicylate de zinc ou de l'un de ses dérivés et d'un sel d'aluminium anti-transpirant tels que définis dans l'une quelconque des revendications précédentes dans un rapport en poids salicylate de zinc /sel d'aluminium allant de 1/500 à 10/1, tels que définis dans l'une quelconque des revendications précédentes comme actif déodorant dans une composition cosmétique.

Office européen des brevets

# RAPPORT DE RECHERCHE EUROPEENNE

Numéro de la demande
EP 06 11 0830

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | EP 1 486 199 A (L'OREAL) 15 décembre 2004 (2004-12-15) * revendications 1,2,1626-28 * * alinéas [0004], [0025] * ----- | 1-32 | INV. A61K8/368 A61Q15/00 A61K8/26 A61K8/27 |
| D,A | EP 0 318 206 A (THE PROCTER & GAMBLE COMPANY) 31 mai 1989 (1989-05-31) * revendications 1,2 * * page 3, ligne 13-20 * ----- | 1-32 | |
| A,D | US 5 254 332 A (GREZCYN ET AL) 19 octobre 1993 (1993-10-19) * colonne 7, ligne 49-60 * ----- | 1-32 | |

DOMAINES TECHNIQUES RECHERCHES (IPC)

A61K

Le présent rapport a été établi pour toutes les revendications

| Lieu de la recherche | Date d'achèvement de la recherche | Examinateur |
|---|---|---|
| Munich | 12 juillet 2006 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................
& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 06 11 0830

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-07-2006

| Document brevet cité au rapport de recherche | Date de publication | Membre(s) de la famille de brevet(s) | Date de publication |
|---|---|---|---|
| EP 1486199 A | 15-12-2004 | FR 2855408 A1 | 03-12-2004 |
| EP 0318206 A | 31-05-1989 | CA 1328408 C | 12-04-1994 |
| | | US 4816261 A | 28-03-1989 |
| US 5254332 A | 19-10-1993 | US 5302381 A | 12-04-1994 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 9301793 A
- EP 468564 A
- EP 024176 A
- EP 768080 A
- EP 318206 A
- US 5254332 A
- US 5302381 A
- WO 0213776 A
- WO 9724131 A
- US 6426061 B
- WO 0152804 A
- EP 1486199 A
- US 3792068 A
- US 4822596 A
- US 4904463 A
- WO 9744010 A

### Littérature non-brevet citée dans la description

- Drugs & Cosmetic Industry-Modern Cosmetics. 1934, 168-177
- **C. FOX.** *Cosmetics and Toiletries,* Novembre 1986, vol. 101, 101-112